# EUROPEAN PATENT APPLICATION

(11) **EP 4 071 232 A1**
(43) Date of publication of application: **12.10.2022**
(21) Application number: 20897171.3
(22) Date of filing: 02.12.2020
(51) Int. Cl.: C12M 1/06, B01F 13/08, B01F 15/00

(54) **BIO-REACTION STIRRER USING MAGNETIC FORCE**

(30) Priority: 02.12.2019 KR 20190158156
(71) Applicant: Kim, Doo-Hyun, Seoul 06503 (KR)
(72) Inventor: Kim, Doo-Hyun, Seoul 06503 (KR)
(74) Representative: Hübner, Gerd
(86) International application number: PCT/KR2020/017435
(87) International publication number: WO 2021/112546

(57) **Abstract**

The present invention relates to a bio-reaction stirrer using a magnetic force. According to an embodiment of the present invention, a driving coupling part is axially formed inside a housing part and a plurality of impellers are provided according to the height direction of the driving coupling part, and the respective impellers can be selectively driven as necessary, thereby efficiently stirring the entire inside of the housing part.

## Description

### Technical Field

The present invention relates to a bio-reaction stirrer using magnetic force.

This application claims priority based on Korean Patent Application No. 10-2019-0158156 filed on December 2, 2019, and all contents described in the specification and drawings of the application are incorporated herein by reference.

### Background Art

Currently, various types of bioreactors including a cell incubator are widely used in a great range of fields including medicine, pharmaceuticals, biotechnology, etc., for the purposes of production of reagents or vaccines, development of new drugs, and treatment and researches using stem cells.

A bioreactor refers to a system that artificially reproduces biochemical reaction processes such as decomposition, synthesis or chemical transformation of substances, etc., in the body of an organism, and is also called a bio-reaction device.

Therefore, depending on the details of various operations performed in the bioreactor, conditions such as nutrients, temperature, humidity, PH, oxygen and carbon dioxide that are suitable for the inside of a container, in which mixing of substances or culturing of cells actually occurs, and stirring conditions should be maintained for a predetermined period so that a biochemical reaction of a sample actively proceeds according to such internal chemical conditions.

On the other hand, Korean Patent Laid-Open Publication No. 10-2010-0083524 (laid-opened on July 22, 2010), as a related art thereto, disclosed "a method for construction of a stirrer in a longitudinal bioreactor and a bioreactor comprising one vertical stirrer".

The above Korean patent laid-open publication has an advantage of promoting the biochemical reaction of a sample inside the bioreactor because a stirring blade is connected to a driving unit through an impeller shaft to rotate by a driving force of the driving unit.

However, the above Korean patent laid-open publication has a disadvantage in that the entire inside of the bioreactor cannot be efficiently stirred because the stirring blade is fixed at a predetermined position inside the bioreactor through the impeller shaft, hence creating a blind spot inside the bioreactor.

### Disclosure

### Technical Problem

As the technical problem to be achieved by the present invention, an object of the present invention is to provide a bio-reaction stirrer capable of efficiently stirring the entire inside of a housing unit, which includes: a drive coupling unit axially formed inside the housing unit; and a plurality of impellers in a height direction of the drive coupling unit, wherein the impellers can be selectively driven as needed.

The object of the present invention is not limited to the above, but other objects not mentioned herein will be clearly understood by persons having ordinary skill in the art ("those skilled in the art") from the following description.

### Technical Solution

In order to achieve the above purposes, one embodiment of the present invention provides a biochemical reaction ("bioreaction") stirrer using magnetic force, including: a housing unit in which a biochemical reaction of a sample occurs inside, and a drive coupling unit formed such that it is recessed toward the inside from one side or penetrates from one side toward the other side; a driving unit provided inside the drive coupling unit, which is provided with a driving magnetic unit whose polarity is changed by a current provided from a power supply unit; and a stirring unit disposed along a periphery of the drive coupling unit inside the housing unit, which includes a driven magnetic unit rotated by the driving magnetic unit and an impeller unit connected to the driven magnetic unit.

Further, with regard to the bioreaction stirrer using magnetic force provided by the present invention, the driven magnetic unit may include: a rotary case provided with the impeller unit on the outside thereof, which is detachably coupled to the drive coupling unit and surrounds the drive coupling unit while being spaced apart from the same at a predetermined interval when coupled to the drive coupling unit; and a driven magnet provided in the rotary case, which rotates together with the rotary case by the polarity of the driving magnetic unit.

Further, with regard to the bioreaction stirrer using magnetic force provided by the present invention, the driven magnetic unit may further include a friction reducing member, which is rotatably provided on an inner peripheral surface of the rotary case, and is rotated in close contact with the drive coupling unit so as to reduce friction with the drive coupling unit when rotating the rotary case.

Further, with regard to the bioreaction stirrer using magnetic force provided by the present invention, the friction reducing member may include: a ball bearing, which is rotatably coupled to the inner peripheral surface of the rotary case, is drawn into the rotary case or drawn out of the inner peripheral surface of the rotary case when the rotary case is coupled to the drive coupling unit, and is rotated in close contact with the drive coupling unit so as to reduce friction between the rotary case and the drive coupling unit when rotating the rotary case; and an elastic member having one end supported by the rotary case and the other end supported by the ball bearing to provide an elastic force whereby the ball bearing is drawn out of the inner peripheral surface of the rotary case to the outside.

### Advantageous effects

According to an embodiment of the present invention, the drive coupling unit is axially formed inside the housing unit, a plurality of impellers are provided in the height direction of the drive coupling unit and the respective impellers can be selectively driven as needed, thereby achieving effects of efficiently stirring the entire inside of the housing unit.

### Description of Drawings

FIG. 1 is a perspective view of a bioreaction stirrer using magnetic force according to an embodiment of the present invention.
FIG. 2 is an exploded perspective view of the bioreaction stirrer using magnetic force shown in FIG. 1.
FIG. 3 is a cross-sectional view showing the internal structure of the bioreaction stirrer using magnetic force shown in FIG. 1.
FIG. 4 is a cross-sectional view and a partially cut-away view for explaining a process of detachably attaching a stirring unit to a drive coupling unit shown in FIG. 2.
FIG. 5 is a block diagram illustrating a schematic configuration of the driving unit shown in FIG. 1.
FIG. 6 is a cross-sectional view of a bioreaction stirrer using magnetic force according to another embodiment of the present invention.
FIGS. 7 and 8 are cross-sectional views illustrating various embodiments of the drive coupling unit shown in FIG. 6.

### Description of numeral symbols

- 10, 20:: Bioreaction stirrer using magnetic force
- 101:: Housing unit 103: Upper housing
- 105:: Lower housing 201: Drive coupling unit
- 203:: Driven magnetic unit 205: Impeller unit
- 207:: Stirring unit 209: Rotational coupling groove
- 211:: Ball bearing 213: First stirring unit
- 215:: Second stirring unit 217: Third stirring unit
- 219:: Rotary case 221: Driven magnet
- 223:: Rotational coupling hole 225: Driven magnet
- 227:: Driven magnet 301: Power supply unit
- 303:: Driving magnetic unit 305: Driving unit
- 307:: Position fixing member 309: Recessed groove
- 311:: Rotational projection 313: Elastic member
- 314:: Concentration measurement unit 315: Control unit
- 317:: Gap 319: First driving magnet
- 321:: Second driving magnet 323: Third driving magnet
- 325:: First measurement part 327: Second measurement part
- 329:: Third measurement part 331: Receiving groove
- 333:: Friction reducing member 335: Elastic member
- 337;: Support member 339: Friction reducing member
- 341:: Friction reducing member 601: Drive coupling unit
- 603:: Restraint guide unit 701: Magnetic field coil
- 801:: Magnetic field coil

### Detailed Description of Preferred Embodiments of Invention

Hereinafter, some embodiments of the present invention will be described in detail with reference to exemplary drawings. With respect to addition of reference numerals to the components of each drawing, it should be noted that the same components are given the same reference numerals as much as possible even though they are indicated on different drawings. Further, in describing the present invention, if it is determined that a detailed description of a related known configuration or function may obscure the gist of the present invention, the detailed description thereof will be omitted.

Further, in describing the components of the present invention, terms such as first, second, A, B, (a), (b), etc. may be used. These terms are only for distinguishing the components from other components, and the essence, sequence and/or order of the components are not limited by the terms. When a component is described as being "bound", "coupled" or "connected" to another component, the component may be directly bound or connected to the other component, but it should be understood that another component (s) may be "bound", "coupled" or "connected" between two components.

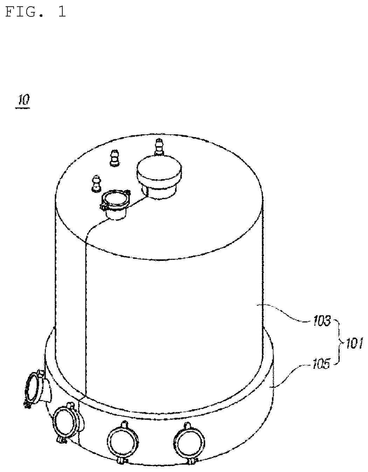

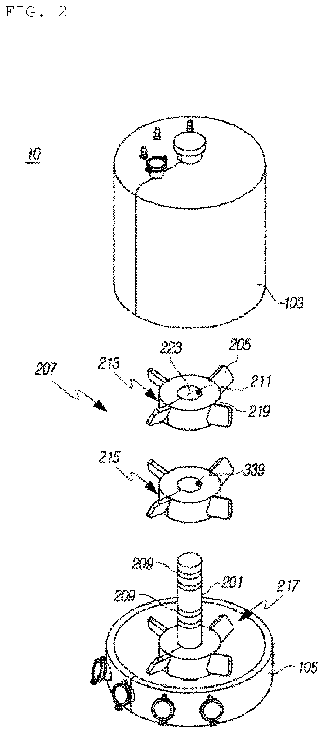

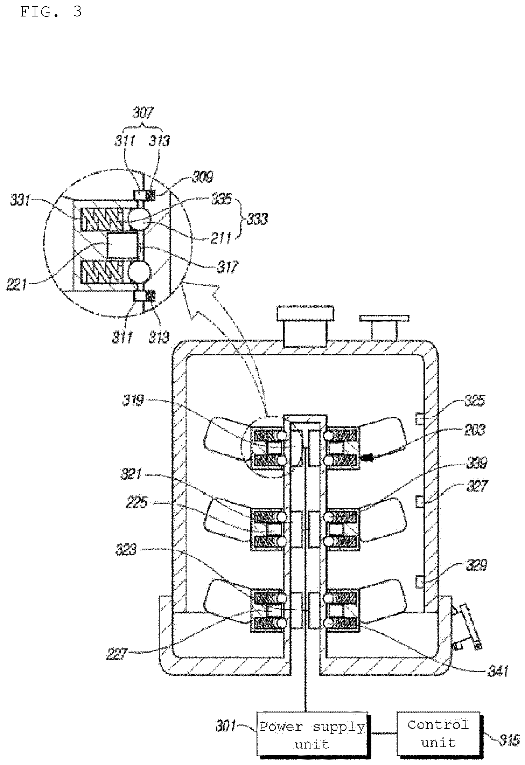

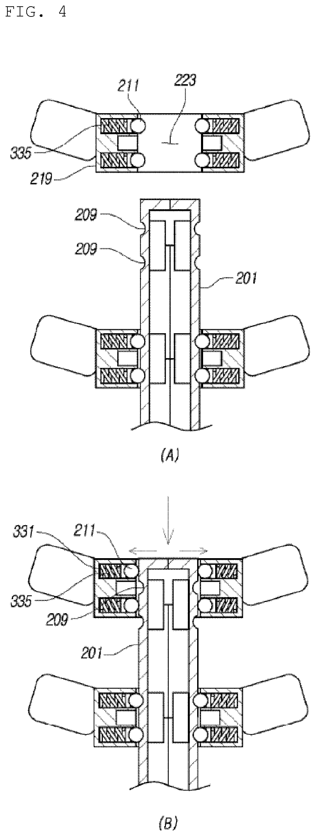

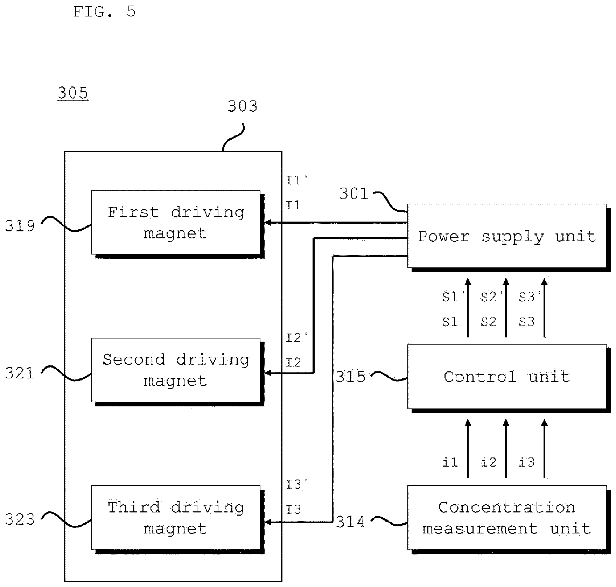

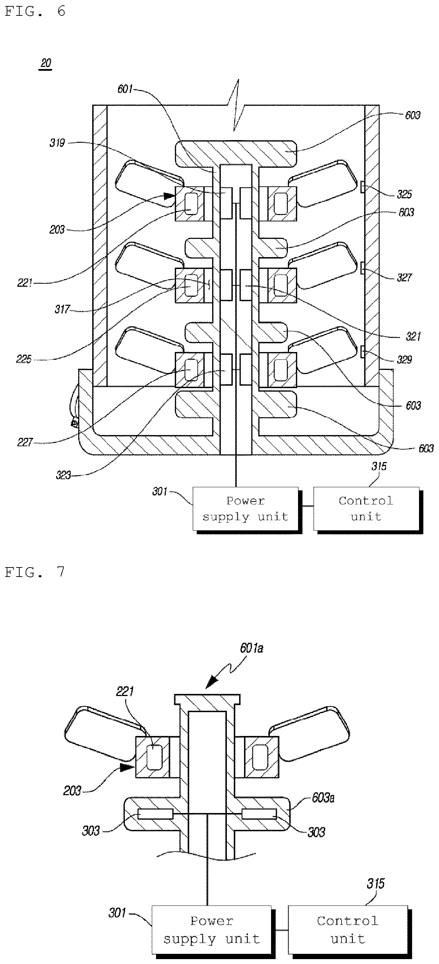

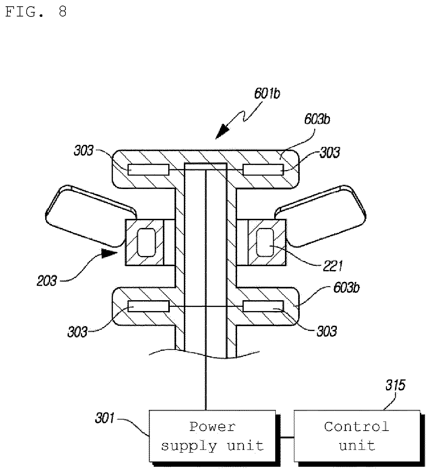

FIG. 1 is a perspective view of a bioreaction stirrer using magnetic force according to an embodiment of the present invention. FIG. 2 is an exploded perspective view of the bioreaction stirrer using magnetic force shown in FIG. 1. FIG. 3 is a cross-sectional view showing the internal structure of the bioreaction stirrer using magnetic force shown in FIG. 1. FIG. 4 is a cross-sectional view and a partially cut-away view for explaining a process of detachably attaching a stirring unit to a drive coupling unit shown in FIG. 2. FIG. 5 is a block diagram illustrating a schematic configuration of the driving unit shown in FIG. 1. FIG. 6 is a cross-sectional view of a bioreaction stirrer using magnetic force according to another embodiment of the present invention. FIGS. 7 and 8 are cross-sectional views illustrating various embodiments of the drive coupling unit shown in FIG. 6.

As shown in these figures, the bioreaction stirrer 10 using magnetic force according to an embodiment of the present invention may include: a housing unit 101 in which a biochemical reaction of a sample occurs inside, and a drive coupling unit 201 is formed such that it is recessed toward the inside from one side or penetrates from one side toward the other side; a driving unit 305 provided inside the drive coupling unit 201, which is provided with a driving magnetic unit 303 whose polarity is changed by a current provided from a power supply unit 301; and a stirring unit 207 disposed along a periphery of the drive coupling unit 201 inside the housing unit 101, which includes a driven magnetic unit 203 rotated by the driving magnetic unit 303 and an impeller unit 205 connected to the driven magnetic unit 203.

The housing unit 101 may include an upper housing 103 provided on an upper portion and a lower housing 103 coupled to the upper housing 103 to form an inner space, whereby the biochemical reaction of a sample occurs inside the housing unit.

Herein, the sample is accommodated in the inner space, and stirring is conducted so as to actively perform the biochemical reaction of the sample.

Further, the housing unit 101 has the drive coupling unit 201 that may be recessed toward the inside from one side or penetrate from one side to the other side. In the drawings, an example of the drive coupling unit 201 recessed toward the inner space from the lower housing 105 is illustrated.

The drive coupling unit 201 may be provided with the driving magnetic unit 303 which is formed in the shape of a cylindrical groove in the lower housing 105, receives power from the power supply unit 301 therein and rotates the stirring unit 207.

Further, the drive coupling unit 201 may have a rotational coupling groove 209 which is recessed in a circumferential direction along an outer peripheral surface thereof.

Such a rotational coupling groove 209 has approximately a hemispherical shape and is recessed in the circumferential direction of the drive coupling unit 201, and may be formed in plural in a longitudinal direction of the drive coupling unit 201 so that a plurality of ball bearings 211 may be coupled thereto.

Moreover, the drive coupling unit 201 may be provided with a recessed groove 309, which is recessed inward in a radial direction, on the outer peripheral surface of the drive coupling unit, wherein the recessed groove 309 has a position fixing member 307 to secure a position of the stirring unit 207.

The position fixing member 307 may include a rotational projection 311 and an elastic member 313, wherein the rotational projection 311 presses the elastic member 313 and is drawn into the recessed groove 309, or a part of the rotational projection is drawn out of the recessed groove 309 to the outside by an elastic force of the elastic member 313.

Further, the rotational projection 311 may be in close contact with an upper surface and a lower surface of each stirring unit 207 to secure a position of the stirring unit 207 in a height direction of the drive coupling unit 201, wherein the rotational projection 311 is provided, for example, in a shaft form extending in a transversal direction and is rotated in close contact with the stirring unit 207 when rotating the stirring unit 207.

The elastic member 313 may be provided in the recessed groove 309, wherein one end is supported by the recessed groove 309 while the other end is supported by the rotational projection 311 to provide the elastic force so that the rotational projection 311 is drawn out of the recessed groove 309 to the outside.

In this way, according to a configuration of providing the position fixing member 308 in that the elastic member 313 is pressed to draw the rotational projection into the recessed groove 309, or the rotational projection is drawn out of recessed groove 309 by the elastic force of the elastic member 313 so as to secure a position of the stirring unit 207, the present invention enables the position of the stirring unit 207 to be secured in the drive coupling unit 201 by drawing the rotational projection 311 into the recessed groove 309 to couple the stirring unit 209 to the drive coupling unit 201 and then drawing the rotational projection 311 out of the recessed groove 309 to the outside.

Further, according to a configuration in that each rotational projection 311 is in close contact with the stirring unit 207 and rotates about a rotation axis formed in the transversal direction, the stirring unit 207 may be secured at a desired position and, at the same time, friction between the stirring unit 207 and the rotational projection 311 may be reduced when rotating the stirring unit 207.

On the other hand, in the case of the bioreaction stirrer 10, after an operation such as cell culture is completed, the bioreaction stirrer 10 can be re-used through a number of procedures, for example, complete removal of residues on the inner space of the housing unit 101 and performing sterilization again. Therefore, for reasons of hygiene, etc., the housing unit 101 may also be provided in the form of a disposable film having flexibility.

Meanwhile, the driving unit 305 may include a driving magnetic unit 303, a power supply unit 301, a concentration measurement unit 314 and a control unit 315.

The driving magnetic unit 303 is provided inside the drive coupling unit 201 in the form of a coil wound several times and thus has polarity changeable by the current supplied from the power supply unit 301. More specifically, the driving magnetic unit 303 may be provided in plural in a circumferential direction inside the drive coupling unit 201 and, when the current is supplied from the power supply unit 301, the polarity may be changed in the circumferential direction and the magnetic force may be applied to the driven magnetic unit 203 to thus rotate the stirring unit 207.

The driving magnetic unit 303 may be provided in plural in the height direction of the drive coupling unit 201. For example, the driving magnetic unit 303 may include a first driving magnet 319, a second driving magnet 321 provided below the first driving magnet 319, and a third driving magnet 323 provided below the second driving magnet 321.

The power supply unit 301 may receive a current control signal transmitted from the control unit 315 and then supply the current to each driving magnetic unit 303 so as to rotate each stirring unit 207.

For example, when a first current control signal S1 is applied from the control unit 315, the power supply unit 301 supplies current I1 to the first driving magnet 319. Further, when a second current control signal S2 is applied from the control unit 315, the power supply unit 310 supplies current I2 to the second driving magnet 321. Further, when a third current control signal S3 is applied from the control unit 315, the power supply unit 310 supplies current T3 to the third driving magnet 323.

The concentration measurement unit 314 may be provided in plural inside the housing unit 101 and spaced apart from one another at a predetermined interval in the height direction of the housing unit 101, and may react with a sample to measure a concentration of the sample and transmit concentration information corresponding to the concentration value of the sample to the control unit 315.

When describing an example of the structure of the concentration measurement unit 314 in more detail, the concentration measurement unit may include a first measurement part 324, a second measurement part 327 and a third measurement part 329, wherein the first measurement part 325 is provided on an upper portion of the housing unit 101 and transmits first concentration information i1 measured by reacting with the sample on the upper portion of the housing unit 101 to the control unit 315.

Further, the second measurement part 327 may be provided inside the housing unit 101, wherein it is disposed between the first measurement part 325 and the third measurement part 329 and transmits second concentration information i2 measured by reacting with the sample present between the first measurement part 325 and the third measurement part 329 to the control unit 315.

Furthermore, the third measurement part 329 may be provided below the second measurement part 327, wherein it transmits third concentration information i3 measured by reacting with the sample present on a lower portion of the housing unit 101 to the control unit 315.

Further, the control unit 315 may generate a current control signal depending upon a preset value and transmit the same to the power supply unit 301 such that the power supply unit 301 supplies a current to the driving magnetic unit 303 according to the preset value so as to selectively rotate one or more stirring units 207.

The control unit 315 may generate a current control signal according to a set value preset ("a preset value") through an input part (not shown) and then transmit the same to the power supply unit 301. For example, the control unit receives first concentration information i1 detected from the first measurement part 325 and, if the first concentration information i1 does not reach a preset value, generates and transmits a first current control signal S1 to the power supply unit 301. At this time, the power supply unit 301 supplies the current to the first driving magnet 319.

Further, the control unit 315 receives second concentration information i2 detected from the second measurement part 325 and, if the second concentration information i2 is less than a preset value, generates and then transmits a second current control signal S2 to the power supply unit 301. At this time, the power supply unit 301 supplies the current to the second driving magnet 321.

Moreover, the control unit 315 receives third concentration information i3 detected from the third measurement part 325 and, if the third concentration information i3 is less than a present value, generates and then transmits a third current control signal S3 to the power supply unit 301. At this time, the power supply unit 301 supplies the current to the third driving magnet 323.

Meanwhile, the control unit 315 receives concentration information (i1, i2, i3) of a sample and, if each concentration information (i1, i2, i3) does not reach a preset value, each of reverse current control signals (S1', S2', S3') may be generated and transmitted to the power supply unit 301 so as to enable a stirring flow to be in the opposite direction. At this time, the power supply unit 301 may generate each of reverse currents (I1', 12', 13') and supply the same to the driving magnetic unit 303, wherein the first driving magnet 319, the second driving magnet 321 and the third driving magnet 323 rotate in opposite directions.

In other words, the control unit 315 receives the concentration information (i1, i2, i3) of the sample corresponding to the height of the housing unit 101 and, if the sample does not reach a preset concentration value, may selectively rotate a plurality of stirring units 207 provided inside the housing unit 101 in the forward or reverse direction, thereby efficiently stirring the sample.

Subsequently, the stirring unit 209 is coupled to the outside of the drive coupling unit 201 and rotated by the driving unit 305.

The stirring unit 209 may be provided in plural in the height direction of the drive coupling unit 201, and the stirring unit 209 may include, for example, a first stirring unit 213, a second stirring unit 215 provided below the first stirring unit 213, and a third stirring unit 217 provided below the second stirring unit 215.

The first stirring unit 213 may include a driven magnetic unit 203 coupled to the outside of the drive coupling unit 201, and an impeller unit 205 provided on the outside of the driven magnetic unit 203.

The driven magnetic unit 203 may include: a rotary case 219 provided with the impeller unit 205 on the outside thereof, which is detachably coupled to the drive coupling unit 201 and surrounds the drive coupling unit 201 while being spaced apart from the same at a predetermined interval when coupled to the drive coupling unit 201; and a driven magnet 221 provided in the rotary case 219, which rotates together with the rotary case 219 by the polarity of the driving magnetic unit 303.

The rotary case 219 is rotatably coupled to the drive coupling unit 201, and more specifically, the rotary case 219 may have a rotational coupling hole 223 formed in the center thereof to penetrate in an axial direction, whereby the rotary case is inserted to the outside of the drive coupling unit 201 through the rotational coupling hole 215.

Herein, the rotational coupling hole 223 may be formed to have a larger diameter than a diameter of the drive coupling unit 201. As such, owing to a structure of the rotational coupling hole 223 with a larger diameter compared to a diameter of the drive coupling unit 201, the rotary case 219 may be spaced apart from the drive coupling unit 201 at a predetermined interval when inserted into the drive coupling unit 201, thereby preventing friction with an outer periphery of the drive coupling unit 201 during rotation.

In other words, since the rotary case 219 has a rotational coupling hole 223 larger than the diameter of the drive coupling unit 201, a gap 317 may occur between the rotary case and the drive coupling unit 201 when inserted into the drive coupling unit 201. As such, owing to a structure in that, when the rotational coupling hole 223 is coupled to the drive coupling unit 201, a gap 317 is formed to prevent friction with the drive coupling unit 201 during rotation, the present invention may prevent a residue, which is generated by friction between the rotary case 219 and the drive coupling unit 201 when rotating the stirring unit 207, from penetrating into the sample.

Further, the rotary case 219 is recessed inward in a radial direction, and may include a plurality of receiving grooves 331 which are formed to be spaced apart from one another at a predetermined interval in the circumferential direction, wherein the receiving groove 331 is provided with a friction reducing member 333 described below.

Subsequently, the driven magnet 221 may rotate together with the rotary case 219 by the polarity of the driving magnetic unit 303.

The driven magnet 221 is provided in the rotary case 219, and more specifically, a plurality of driven magnets may be arranged at equal intervals in the circumferential direction inside the rotary case.

The driven magnet 221 rotates in the circumferential direction of the drive coupling unit 201 by magnetic force of a first driving magnet 319 when the polarity of the first driving magnet 319 is changed in the circumferential direction, thereby rotating together with the rotary case 219.

On the other hand, the driven magnetic unit 203 according to an embodiment of the present invention may further include a friction reducing member 333, which is rotatably provided on an inner peripheral surface of the rotary case 219, is rotated in close contact with the drive coupling unit 201 so as to reduce friction with the drive coupling unit 201 when rotating the rotary case 219.

Such a friction reducing member 333 may be provide in plural and arranged on the inner peripheral surface of the rotary case 219 while being spaced apart from one another at a predetermined interval. When describing an example of a structure of the friction reducing member 333 in more detail, the friction reducing member 333 may include: a ball bearing 211, which is rotatably coupled to the inner peripheral surface of the rotary case 219, is drawn into the rotary case 309 or drawn out of the inner peripheral surface of the rotary case 219 to the outside when the rotary case 219 is coupled to the drive coupling unit 201, and is rotated in close contact with the drive coupling unit 201 so as to reduce friction between the rotary case 219 and the drive coupling unit 201 when rotating the rotary case 219; and an elastic member 335 having one end supported by the rotary case 219 and the other end supported by the ball bearing 211 to provide an elastic force, whereby the ball bearing can be drawn out of the inner peripheral surface of the rotary case 219 to the outside.

The ball bearing 211 may be provided to be drawn into or out of the receiving groove 331 of the rotary case 219, wherein, when the rotary case 219 is coupled to the drive coupling unit 210, the ball bearing is coupled in the rotational coupling groove 209 formed in the drive coupling unit 201.

Further, the ball bearing 211 receives an elastic force through the elastic member 335 and is drawn out of the inner peripheral surface of the rotary case 219 to the outside. At this time, a portion of the ball bearing is rotatably in close contact with a support member 337 provided between the ball bearing and the elastic member 335 so as to reduce friction with the elastic member 335 during rotation while being in close contact with the rotational coupling groove 209.

In this case, the support member 337 may be provided to be movable into the receiving groove 331, wherein the support member is disposed between the ball bearing 335 and the elastic member 335 to transfer an elastic force imparted from the elastic member 335 to the ball bearing 335, and one side thereof is recessed to correspond to the ball bearing 211, whereby the recessed side is rubbed with the ball bearing 335 when rotating the ball bearing 335.

Referring to FIG. 4, a process of coupling the rotary case 219 with the drive coupling unit 201 through the friction reducing member 333 will be described in more detail. First, when the rotary case 219 is separated from the drive coupling unit 201, the ball bearing 211 receives an elastic force from the elastic member 355 and thus is drawn out of the inner peripheral surface of the rotary case 219 to the outside, as shown in FIG. 4(A).

In this regard, when the rotary case 223 is coupled with the drive coupling unit 201 by an external force, the ball bearing 211 is in close contact with the drive coupling unit 201 to press the elastic member 335, which in turn is drawn into the receiving groove 331. Following this, the rotary case 223 is inserted along the drive coupling unit 201 and, when located at a predetermined position of the drive coupling unit 201, a portion of the ball bearing 211 is inserted into the rotational coupling groove 209 to secure the stirring unit 207 on a predetermined height of the drive coupling unit 201.

At this time, when the rotary case 219 rotates, the ball bearing 211 rotates along a periphery of the drive coupling unit 201 together with the rotary case 219 and, at the same time, is in close contact with the rotational coupling groove 209 so as to reduce friction between the rotary case 219 and the drive coupling unit 201 while rotating inside the receiving groove 331.

In this way, owing to a configuration of providing the friction reducing member 333 which is drawn into or out of the receiving groove 331 and is rotated in close contact with the drive coupling unit 201, the present invention can easily attach/detach the stirring unit 207 to/from the housing unit 101 as needed, and can reduce friction between the stirring unit 207 and the housing unit 101 when stirring a sample.

On the other hand, the second stirring unit 215 may be provided with an impeller unit on the outside thereof like the firs stirring unit 213, and may also be provided with a driven magnet 225 rotated by the second driving magnet 321 and a friction reducing member 339 therein.

Further, the third stirring unit 217 may be provided with an impeller unit on the outside thereof like the first and second stirring units 213 and 215, and may also provided with a driven magnet 227 rotated by the third driving magnet 323 and a friction reducing member 341 therein.

Among the above stirring units 207, the first stirring unit 325 is provided inside the housing unit 101 at a site corresponding to a first measurement part 325, the second stirring unit 215 is provided inside the housing unit 101 at a site corresponding to a second measurement part 327, and the third stirring unit 217 is provided inside the housing unit 101 at a site corresponding to a third measurement part 329.

As such, a plurality of stirring units 207 may be provided at corresponding positions of the first measurement part 325, the second measurement part 327 and the third measurement part 329, respectively so that, when a concentration of the sample measured through each of the measurement parts 325, 327 and 329 is less than a set value, the sample can be efficiently stirred by selectively rotating the stirring units 207 corresponding to positions of the measurement parts 325, 327 and 329, respectively.

Since the second stirring unit 215 and the third stirring unit 217 have the same structure and effects as the first stirring unit 213, it is of course construed that subject matters not described in regard to the second stirring unit 215 and the third stirring unit 217 could be understood from the structure and effects of the first stirring unit 213.

On the other hand, referring to FIG. 6, the bioreaction stirrer 20 using magnetic force according to another embodiment of the present invention may comprise the same structures as the bioreaction stirrer 10 using magnetic force according to one embodiment of the present invention except for some components.

For example, the friction reducing member 333 is removed from the driven magnetic unit 203.

Further, an installation site of the driving magnetic unit 303 may be altered.

Further, the drive coupling unit 601 may comprise a structure different from that of the drive coupling unit 201 according to the previous embodiment of the present invention.

When describing the structure of the drive coupling unit 601 in more detail, the drive coupling unit 601 may have a plurality of restraint guide units 603 formed on upper and lower sides of each driven magnetic unit 203.

The restraint guide unit 603 is formed to protrude in the circumferential direction of the drive coupling unit 601 from the upper and lower sides of the driven magnetic unit 203, and may have a function of preventing detachment (or release) of the driven magnetic unit 203 from the drive coupling unit 601 when rotating the driven magnetic unit 203.

As such, according to another embodiment of the present invention, a gap is formed between the drive coupling unit 601 and the driven magnetic unit 203 so as to minimize friction generated during stirring, as well as to prevent release of the driven magnetic unit 203 from the drive coupling unit 601 through the restraint guide unit 603.

On the other hand, the above-described drive coupling unit 601 may be configured in various embodiments.

When describing another embodiment of the drive coupling unit 601a with reference to FIG. 7, the drive coupling unit 601a may have a restraint guide unit 603a formed on the lower side of the driven magnetic unit 203, and a driving magnetic unit 303 provided inside the restraint guide unit 603a.

Herein, as described above, the driving magnetic unit 303 may be provided in the form of a coil wound therein, whose polarity is changed in the circumferential direction by the current supplied from the power supply unit 301. As such, the driving magnetic unit 303 receives power from the power supply unit 301 and forms a magnetic force, thereby rotating the stirring unit 207.

In this way, since the driving magnetic unit 303 is provided inside the restraint guide unit 603a, this embodiment of the present invention may supply a magnetic force generated in a wider area compared to other embodiments to the driven magnet 221, thereby improving stirring efficiency.

Herein, as shown in FIG. 6, when a plurality of driven magnetic units 203 is provided, it is of course possible that the restraint guide unit 603a is also provided in plural below the driven magnetic units 202, respectively.

Meanwhile, referring to FIG. 8, another embodiment of the drive coupling unit 601b will be described. Specifically, the drive coupling unit 601b may include restraint guide units 603b formed on upper and lower sides of the driven magnetic unit 203.

Each of the restraint guide units 603b may be provided with a driving magnetic unit 303 therein, which receives power from the power supply unit 301 and forms a magnetic force.

In this way, since the restraint guide units 603b are formed on the upper and lower sides of the driven magnetic unit 203, and the driving magnetic unit 303 is provided inside each of the restraint guide unit 603b, this embodiment of the present invention may supply the magnetic force to the driven magnet 221 from both of the upper and lower sides of the driven magnetic unit 203, respectively, thereby improving stirring efficiency.

Moreover, even when the driven magnetic unit 203 is spaced from the drive coupling unit 601b at a predetermined interval, it can be rotated stably without being separated from the drive coupling unit 601b.

Meanwhile, when a plurality of driven magnetic units 203 is provided as shown in FIG. 6, the restraint guide unit 603b may also be provided in plural on upper and lower sides of the driven magnetic units 203, respectively.

As described above, according to the embodiments of the present invention, the drive coupling unit 201 is formed in an axial direction inside the housing unit 101, a plurality of stirring units (213, 215, 217) is provided in a height direction of the drive coupling unit 201, and the respective impellers may be selectively driven as needed, whereby the entire inside of the housing unit 101 can be efficiently stirred.

In the above, preferred embodiments of the present invention have been illustrated and described, however, the present invention is not limited to such specific and preferred embodiments as described above. Therefore, anyone skilled in the technical field to which the present invention pertains would of course implement various modifications without departing from the gist of the present invention as claimed in the appended claims, and such alterations are within the scope of the claims.

## Claims

1. A biochemical reaction ("bioreaction") stirrer using magnetic force, comprising:
a housing unit in which a biochemical reaction of a sample occurs inside, and a drive coupling unit formed such that it is recessed toward an inside from one side or penetrates from one side toward another side;
a driving unit provided inside the drive coupling unit, which is provided with a driving magnetic unit whose polarity is changed by a current provided from a power supply unit; and
a stirring unit disposed along a periphery of the drive coupling unit inside the housing unit, which includes a driven magnetic unit rotated by the driving magnetic unit and an impeller unit connected to the driven magnetic unit.

2. The bioreaction stirrer according to claim 1, wherein the driven magnetic unit includes:
a rotary case provided with the impeller unit on an outside thereof, which is detachably coupled to the drive coupling unit and surrounds the drive coupling unit while being spaced apart from the same at a predetermined interval when coupled to the drive coupling unit; and
a driven magnet provided in the rotary case, which rotates together with the rotary case by the polarity of the driving magnetic unit.

3. The bioreaction stirrer according to claim 2, wherein the driven magnetic unit further includes:
a friction reducing member which is rotatably provided on an inner peripheral surface of the rotary case, and is rotated in close contact with the drive coupling unit so as to reduce friction with the drive coupling unit when rotating the rotary case.

4. The bioreaction stirrer according to claim 3, wherein the friction reducing member includes:
a ball bearing, which is rotatably coupled to the inner peripheral surface of the rotary case, is drawn into the rotary case or drawn out of the inner peripheral surface of the rotary case to an outside when the rotary case is coupled to the drive coupling unit, and is rotated in close contact with the drive coupling unit so as to reduce friction between the rotary case and the drive coupling unit when rotating the rotary case; and
an elastic member having one end supported by the rotary case and another end supported by the ball bearing to provide an elastic force, whereby the ball bearing is drawn out of the inner peripheral surface of the rotary case to the outside.

5. The bioreaction stirrer according to claim 1, wherein the stirring unit is provided in plural in a height direction of the drive coupling unit.

6. The bioreaction stirrer according to claim 5, wherein the driving unit further includes,
a control unit which generates a current control signal depending upon a preset value and transmits the same to the power supply unit such that the power supply unit supplies a current to the driving magnetic unit according to the preset value so as to selectively rotate one or more stirring units.
